(19) Europäisches Patentamt
European Patent Office
Office européen des brevets

(11) Veröffentlichungsnummer: **0 505 802 A1**

# (12) EUROPÄISCHE PATENTANMELDUNG

(21) Anmeldenummer: **92103954.1**

(22) Anmeldetag: **07.03.92**

(51) Int. Cl.[5]: **C07D 211/46**, C08K 5/00, C08L 23/00

(30) Priorität: **23.03.91 DE 4109650**

(43) Veröffentlichungstag der Anmeldung: **30.09.92 Patentblatt 92/40**

(84) Benannte Vertragsstaaten: **CH DE FR GB IT LI**

(71) Anmelder: **BASF Aktiengesellschaft**
**Carl-Bosch-Strasse 38**
**W-6700 Ludwigshafen(DE)**

(72) Erfinder: **Aumueller, Alexander, Dr.**
**Rieslingweg 25**
**W-6730 Neustadt(DE)**
Erfinder: **Trauth, Hubert**
**Milanstrasse 6**
**W-6724 Dudenhofen(DE)**

(54) **Cyclische beta-Ketocarbonsäure-polyalkylpiperidinylester.**

(57) Cyclische β-Ketocarbonsäure-polyalkylpiperidinylester I

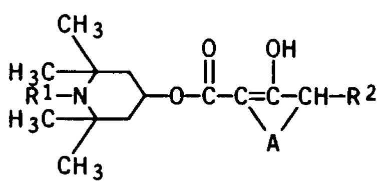

I

in der

- $R^1$ Wasserstoff, $C_1$- bis $C_8$-Alkyl, Phenyl- oder Tolylalkyl mit 1 bis 4 C-Atomen in der Alkylgruppe, $C_1$- bis $C_6$-Acyl, Benzoyl, Allyl, Cyanmethyl, Hydroxyethyl, Aminoethyl, Hydroxyl oder das Oxyl-Radikal bezeichnet,
- $R^2$ für Wasserstoff oder $C_1$- bis $C_4$-Alkyl steht und
- A eine geradkettige oder verzweigte Alkylenbrücke aus 2 bis 10 C-Atomen, wobei hierdurch ein fünf-, sechs- oder siebengliedriger Ring ausgebildet wird, oder eine Gruppierung der Formel

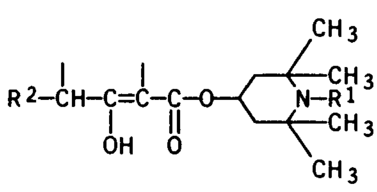

bedeutet,

sowie Tautomere und Säureadditionssalze der Verbindungen I.

Die Verbindungen I dienen als Lichtschutzmittel und Stabilisatoren für organisches Material, insbesondere für Kunststoffe und Lacke.

EP 0 505 802 A1

Die vorliegende Erfindung betrifft neue cyclische β-Ketocarbonsäure-polyalkylpiperidinylester der allgemeinen Formel I

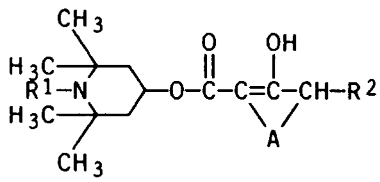

I

in der

$R^1$ Wasserstoff, $C_1$- bis $C_8$-Alkyl, Phenyl- oder Tolylalkyl mit 1 bis 4 C-Atomen in der Alkylgruppe, $C_1$- bis $C_6$-Acyl, Benzoyl, Allyl, Cyanmethyl, Hydroxyethyl, Aminoethyl, Hydroxyl oder das Oxyl-Radikal bezeichnet,

$R^2$ für Wasserstoff oder $C_1$- bis $C_4$-Alkyl steht und

A eine geradkettige oder verzweigte Alkylenbrücke aus 2 bis 10 C-Atomen, wobei hierdurch ein fünf-, sechs- oder siebengliedriger Ring ausgebildet wird, oder eine Gruppierung der Formel

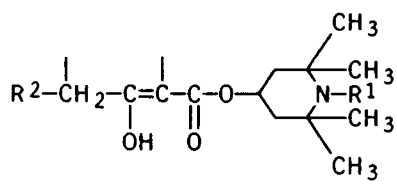

bedeutet,

sowie Tautomere und Säureadditionssalze der Verbindungen I.

Weiterhin betrifft die Erfindung mit den Verbindungen I gegen die Einwirkung von Licht, Sauerstoff und Warme stabilisiertes organisches Material, insbesondere stabilisierte Kunststoffe und Lacke.

Organisches Material, insbesondere Kunststoffe und Lacke, wird bekanntermaßen sehr schnell, vor allem durch Einwirkung von Licht, zerstört. Diese Zerstörung zeigt sich üblicherweise in Vergilbung, Verfärbung, Rißbildung oder Versprödung des Materials. Mit Lichtschutzmitteln und Stabilisatoren soll daher ein zufriedenstellender Schutz gegen die Zerstörung von organischem Material durch Licht, Sauerstoff und Wärme erzielt werden.

So werden in der EP-A 000 487 Enolgruppen enthaltende Verbindungen und deren Metallchelate als Lichtschutzmittel insbesondere in hochmolekularen Verbindungen empfohlen. Als Beispiele werden u.a. 2,2,6,6-Tetramethyl- und 1,2,2,6,6-Pentamethyl-4-(1',3'-dioxobutyloxy)-piperidin sowie deren Nickel-, Magnesium- oder Aluminiumkomplex genannt.

Unbefriedigend ist bei derartigen Mitteln des Standes der Technik häufig die geringe Verträglichkeit mit Kunststoffen, z.B. Polyolefinen, die geringe Dauer der Schutzwirkung, die Eigenfarbe der Substanzen, die Neigung zur Flüchtigkeit und die thermische Zersetzung der Stabilisatoren beim Einarbeiten bei erhöhter Temperatur.

Aufgabe der vorliegenden Erfindung war es daher, Lichtschutzmittel bzw. Stabilisatoren bereitzustellen, die einen wirkungsvolleren Schutz für organisches Material mit sich bringen.

Demgemäß wurden die eingangs definierten cyclischen β-Ketocarbonsäurepolyalkylpiperidinylester I gefunden.

Für $R^1$ kommen neben Wasserstoff vor allem Methyl, Ethyl, Propyl, Butyl, Pentyl, Hexyl, Benzyl, β-Phenylethyl, γ-Phenylpropyl, o-, m- oder p-Methylbenzyl, Allyl, Acetyl, Propionyl, Butanoyl, Pentanoyl, Benzoyl, Cyanmethyl, β-Hydroxyethyl, β-Aminoethyl und Hydroxyl sowie das Oxyl-Radikal in Betracht. Davon werden Methyl, Acetyl, Cyanomethyl, β-Aminoethyl und insbesondere Wasserstoff bevorzugt.

$R^2$ steht für $C_1$- bis $C_4$-Alkyl, z.B. Methyl, Ethyl, Propyl oder Butyl, oder vorzugsweise für Wasserstoff.

Als geradkettige oder verzweigte Alkylenbrücken A eignen sich vor allem 1,2-Ethylen-, 1,3-Propylen- und 1,4-Butylengruppen, welche als Seitenketten Alkylreste wie Methyl, Ethyl, Propyl oder Butyl tragen können, z.B. 1,2-Propylen, 1,2-Butylen, 2,3-Butylen, 1,3-Butylen, 2-Methyl-1,3-propylen, 2-Butyl-1,3-propylen, 1-Methyl-1,4-butylen oder 2-Methyl-1,4-butylen. Bevorzugt werden für A Polymethylenbrücken der Formel $-(CH_2)_n-$, wobei n für 2, 3 oder 4, insbesondere für 3, steht.

Verbindungen I, bei denen $R^2$ für Wasserstoff steht und A gleichzeitig eine derartige Polymethylenbrükke der Formel $(CH_2)_n$- bedeutet, sind besonders bevorzugt.

Weiterhin werden cyclische $\beta$-Ketocarbonsäure-polyalkylpiperidinylester bevorzugt, bei denen A eine Gruppierung der Formel

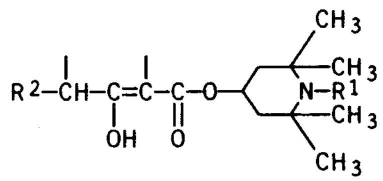

bedeutet. Bei solchen Strukturen handelt es sich um verdoppelte cyclische $\beta$-Ketocarbonsäure-polyalkylpiperidinylester. Insbesondere sind Strukturen der Formel Ia

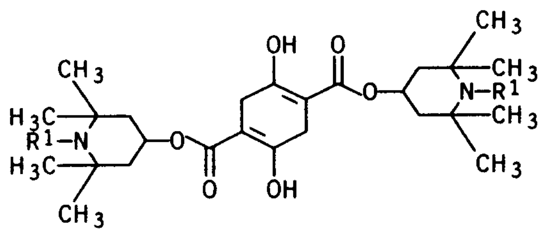

Ia

von Interesse.

Die Verbindungen I werden zweckmäßigerweise durch Umesterung von cyclischen Carbonsäureestern niederer Alkohole wie den Verbindungen der allgemeinen Formeln II und IIa

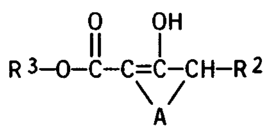

II

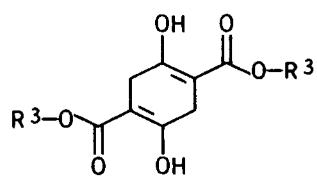

IIa

in denen $R^3$ vor allem für Methyl oder Ethyl, daneben aber für n-Propyl, iso-Propyl, n-Butyl oder tert.-Butyl steht, mit Polyalkylpiperidinolen der allgemeinen Formel III

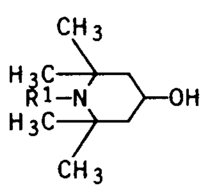

III

in an sich bekannter Weise hergestellt.

Die Umsetzung läßt sich sowohl mit als auch ohne ein inertes organisches Lösungsmittel bei erhöhter Tempratur, etwa bei 80 bis 200 °C, durchführen. Als Lösungsmittel kann dabei auch ein Überschuß einer der beiden Reaktanden fungieren. Der entstehende Alkohol $R^3$-OH wird üblicherweise abdestilliert.

Zweckmäßigerweise verwendet man bei der Umesterung einen Katalysator, vornehmlich eine Protonensäure oder eine Lewissäure, in den hierbei üblichen Mengen. Als Beispiele seien hierfür genannt: Saure Ionenaustauscher auf der Basis von polymeren aromatischen Sulfonsäuren, Titanalkoholate wie z.B. Tetramethylorthotitanat oder Tetrabutylorthotitanat, Eisen-III-chlorid, para-Toluolsulfonsäure, insbesondere jedoch Zink-II-chlorid und vor allem Borsäure.

Die erfindungsgemäßen Verbindungen I können in an sich bekannter Weise, insbesondere durch Destillation oder Kristallistion, isoliert und gereinigt werden.

Je nach Substituenten und Lösungsmitteln können die Verbindungen I auch ganz oder teilweise in tautomeren Formen vorliegen; so gilt etwa das folgende Gleichgewicht:

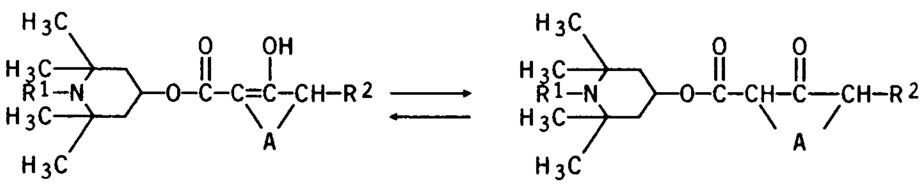

Die erfindungsgemäßen Verbindungen I können in Form der freien Basen oder als Säureadditionssalze vorliegen. Geeignete Anionen stammen z.B. von anorganischen Säuren und insbesondere von organischen Carbonsäuren sowie organischen Sulfonsäuren.

Als anorganische Anionen sind z.B. Chlorid, Bromid, Sulfat, Dicyanimid, Methosulfat, Tetrafluoroborat, Phosphat und Rhodanid zu nennen.

Als Carbonsäureanionen kommen z.B. Formiat, Acetat, Propionat, Hexanoat, Cyclohexanoat, Lactat, Stearat, Dodecylbenzoat, Acrylat, Methacrylat, Citrat, Malonat oder Succinat sowie Anionen von Polycarbonsäuren mit bis zu 3000 COOH-Gruppen in Betracht.

Sulfonsäure-Anionen sind beispielsweise Benzolsulfonat oder Tosylat.

Die erfindungsgemäßen cyclischen $\beta$-Ketocarbonsäure-polyalkylpiperidinylester I eignen sich in hervorragender Weise zum Stabilisieren von organischem Material gegen die Einwirkung von Licht, Sauerstoff und Wärme. Sie sind auch wirksam als Metalldesaktivator. Sie werden dem zu stabilisierenden organischen Material in einer Konzentration von 0,01 bis 5 Gew.-%, vorzugsweise von 0,02 bis 1 Gew.-%, bezogen auf das organische Material, vor, während oder nach seiner Herstellung zugesetzt.

Unter organischem Material sind beispielsweise kosmetische Präparate wie Salben und Lotionen, Arzneimittelformulierungen wie Pillen und Zäpfchen oder Vorprodukte für Kunststoffe und Lacke, insbesondere jedoch Kunststoff und Lacke selbst, zu verstehen.

Gegenstand der vorliegenden Erfindung ist außerdem gegen die Einwirkung von Licht, Sauerstoff und Wärme stabilisiertes organisches Material, insbesondere Kunststoff und Lacke, welches eine oder mehrere der Verbindungen 1 in den oben angegebenen Konzentrationen enthält.

Zur Vermischung der erfindungsgemäßen Verbindungen I vor allem mit Kunststoffen können alle bekannten Vorrichtungen und Methoden zum Einmischen von Stabilisierungsmitteln oder anderen Zusätzen in Polymere angewandt werden.

Das durch die erfindungsgemäßen Verbindungen I stabilisierte organische Material kann gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-$\beta$-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[$\beta$-(3,5-di-tert.-butyl-4-hydroxyphenyl)-propionylethyl]isocyanurat, 1,3,5-Tris-(2,6-dimethyl-3-hydroxy-4-tert.-butylbenzyl)-isocyanuratund Pentaerythrit-tetrakis-[$\beta$-(3,5-di-tert.-butyl-4-hydroxy-phenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-($\beta$-laurylthiopropionat) und Pentaerythrittetrakis-($\beta$-hexylthiopropionat) genannt.

Weitere Antioxidantien und Lichtstabilisatoren, die zusammen mit den erfindungsgemäßen Verbindungen verwendet werden können, sind z.B. 2-(2'-Hydroxyphenyl)-benztriazole, 2-Hydroxybenzophenone, Arylester von Hydroxybenzoesäuren, $\alpha$-Cyanozimtsäurederivate, Benzimidazolcarbonsäureanilide, Nickelverbin-

dungen oder Oxalsäuredianilide.

Als Kunststoffe, die durch die erfindungsgemäßen Verbindungen I stabilisiert werden können, seien beispielsweise genannt:

Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methylacrylat, Acrylnitril-Butadien-Styrol (ABS) oder Methylmethacrylat-Butadien-Styrol (MBS);

halogenhaltige Polymere, wie z.B. Polyvinylchlorid, Polyvinylfluorid, Polyvinylidenfluorid sowie deren Copolymere;

Polymere, die sich von α,β-ungesättigten Säuren und deren Derivaten ableiten, wie Polyacrylate, Polymethacrylate, Polyacrylamide und Polyacrylnitrile;

Polymere, die sich von ungesättigten Alkoholen und Aminen bzw. von deren Acrylderivaten oder Acetalen ableiten, z.B. Polyvinylalkohol und Polyvinylacetat;

Polyurethane, Polyamide, Polyharnstoffe, Polyester, Polycarbonate, Polysulfone, Polyethersulfone und Polyetherketone.

Weiterhin können mit den erfindungsgemäßen Verbindungen I Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die erfindungsgemäßen Verbindungen I können in fester oder gelöster Form dem Lack zugesetzt werden. Ihre gute Löslichkeit in Lacksystemen ist dabei von besonderem Vorteil.

Bevorzugt werden die erfindungsgemäßen Verbindungen I zum Stabilisieren von SAN-Polymeren und insbesondere von ABS-Polymeren verwendet.

Ein weiteres bevorzugtes Einsatzgebiet ist die Stabilisierung von Polyolefinen, insbesondere von Polypropylen.

Die erfindungsgemäßen Verbindungen I zeichnen sich durch eine gute Verträglichkeit mit den üblichen Kunststoffarten und durch eine gute Löslichkeit in den üblichen Lacksystemen aus. Sie haben in der Regel keine oder nur eine sehr geringe Eigenfarbe, sind bei den üblichen Kunststoff- und Lack-Verarbeitungstemperaturen stabil und nicht flüchtig und bewirken vor allen Dingen eine lange Schutzdauer der mit ihnen behandelten Materialien.

Die Erfindung wird durch die nachstehenden Beispiele weiter erläutert. Die Herstellbedingungen wurden nicht optimiert.

Herstellungsbeispiele

Beispiel 1

Eine Mischung aus 157 g (1,0 mol) 2,2,6,6-Tetramethylpiperidin-4-ol, 255 g (1,5 mol) Cyclohexanon-2-carbonsäureethylester und 10 g Borsäure wurde zuerst 4 h auf 150 °C und dann 6 h auf 195 °C erhitzt, wobei Ethanol abdestillierte. Nach Abkühlung verdünnte man mit 1,5 l n-Hexan, filtriert das überrschüssige ausgefallene 2,2,6,6-Tetramethylpiperidin-4-ol (61 g) ab, engte das Hexan-Filtrat ein und destillierte den Rückstand fraktionierend im Vakuum. Man erhielt bei einem Siedepunkt von 155 °C und einem Druck von 0,3 mbar 100,2 g (entsprechend einer Ausbeute von 58 %) der Verbindung der Formel

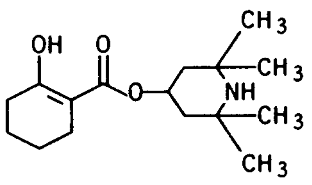

als farbloses öl, das nach einigen Stunden Stehenlassen erstarrt (Schmp.: 60 °C)

| Analyse | | | | |
|---|---|---|---|---|
| Ber.: | C 68,3 | H 9,7 | N 5,0 | O 17,1 |
| Gef.: | C 68,2 | H 9,8 | N 5,0 | O 17,0 |

Beispiel 2

Eine Mischung aus 38,4 g (0,15 mol) 2,5-Dihydroxy-1,4-cyclohexadien-1,4-dicarbonsäurediethylester, 51,3 g (0,3 mol) 1,2,2,6,6-Pentamethylpiperidin-4-ol und 3 g Borsäure wurde 6 h auf 145°C erhitzt, wobei Ethanol abdestillierte. Nach Abkühlung gab man 250 ml einer Mischung aus Ethanol und Wasser in Gew.-Verhältnis von 1 : 1 zu und saugte den ausgefallenen Niederschlag ab. Nach dem Trocknen erhielt man 41,2 g (entsprechend einer Ausbeute von 54 %) der Verbindung der Formel

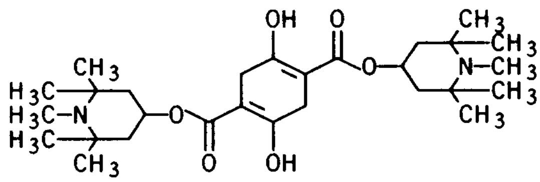

als farblosen Feststoff vom Schmp. 180 bis 182°C. Durch Auskochen mit Ethanol konnte das Produkt weiter gereinigt werden, der Schmp. stieg auf 210 bis 212°C.

| Analyse | | | | |
|---|---|---|---|---|
| Ber.: | C 66,5 | H 9,0 | N 5,5 | O 19,0 |
| Gef.: | C 66,3 | H 9,1 | N 5,4 | O 18,9 |

Beispiel 3

Eine Mischung aus 256 g (1,0 mol) 2,5-Dihydroxy-1,4-cyclohexadien-1,4-dicarbonsäurediethylester, 314 g (2,0 mol) 2,2,6,6-Tetramethyl-piperidin-4-ol und 10 g Borsäure wurde 3,5 h auf 170°C erhitzt, wobei Ethanol abdestillierte. Man kühlte auf 90°C ab, gab 1 l Wasser zu und saugte den entstandenen Niederschlag noch heiß ab. Nach dem Trocknen im Vakuum erhielt man 295 g (entsprechend einer Ausbeute von 62 %) der Verbindung der Formel

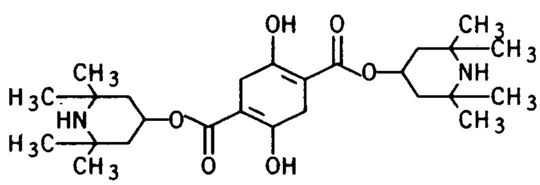

als farblosen Feststoff vom Schmp. 236 bis 239°C. Nach Umkristallisation aus Toluol stieg der Schmp. auf 239 bis 242°C an.

| Analyse | | | | |
|---|---|---|---|---|
| Ber.: | C 65,2 | H 8,8 | N 5,9 | O 20,1 |
| Gef.: | C 65,3 | H 9,0 | N 5,8 | O 20,0 |

**Patentansprüche**

**1.** Cyclische $\beta$-Ketocarbonsäure-polyalkylpiperidinylester der allgemeinen Formel I

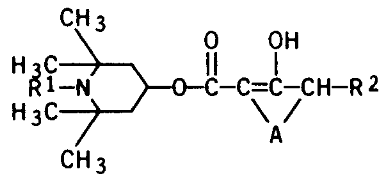

I

in der

$R^1$ Wasserstoff, $C_1$- bis $C_8$-Alkyl, Phenyl- oder Tolylalkyl mit 1 bis 4 C-Atomen in der Alkylgruppe, $C_1$- bis $C_6$-Acyl, Benzoyl, Allyl, Cyanmethyl, Hydroxyethyl, Aminoethyl, Hydroxyl oder das Oxyl-Radikal bezeichnet,

$R^2$ für Wasserstoff oder $C_1$- bis $C_4$-Alkyl steht und

A eine geradkettige oder verzweigte Alkylenbrücke aus 2 bis 10 C-Atomen, wobei hierdurch ein fünf-, sechs- oder siebengliedriger Ring ausgebildet wird, oder eine Gruppierung der Formel

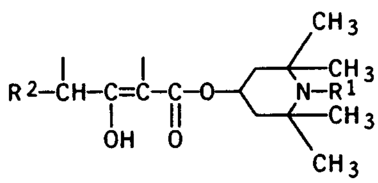

bedeutet,

sowie Tautomere und Säureadditionssalze der Verbindungen I.

**2.** Cyclische $\beta$-Ketocarbonsäure-polyalkylpiperidinylester I nach Anspruch 1, bei denen $R^1$ Methyl, Acetyl, Cyanomethyl, $\beta$-Aminoethyl oder Wasserstoff bezeichnet.

**3.** Cyclische $\beta$-Ketocarbonsäure-polyalkylpiperidinylester I nach Anspruch 1 oder 2, bei denen $R^2$ für Wasserstoff steht und A eine Polymethylenbrücke der Formel $-(CH_2)_n-$ bedeutet, wobei n für 2, 3 oder 4 steht.

**4.** Cyclische $\beta$-Ketocarbonsäure-polyalkylpiperidinylester I nach Anspruch 1 oder 2, bei denen A eine Gruppierung der Formel

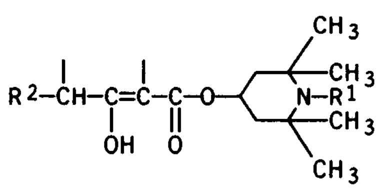

bedeutet.

**5.** Gegen die Einwirkung von Licht, Sauerstoff und Wärme stabilisiertes organisches Material, enthaltend 0,01 bis 5 Gew.-%, bezogen auf die Menge des organischen Materials, eines oder mehrerer cyclischer $\beta$-Ketocarbonsäure-polyalkylpiperidinylester I gemäß den Ansprüchen 1 bis 4.

**6.** Gegen die Einwirkung von Licht, Sauerstoff und Wärme stabilisierte Kunststoffe und Lacke, enthaltend 0,01 bis 5 Gew.-%, bezogen auf die Menge der Kunststoffe bzw. Lacke, eines oder mehrerer cyclischer $\beta$-Ketocarbonsäure-polyalkylpiperidinylester I gemäß den Ansprüchen 1 bis 4.

**7.** Verwendung von cyclischen $\beta$-Ketocarbonsäure-polyalkylpiperidinylestern I gemäß den Ansprüchen 1 bis 4 als Lichtschutzmittel und Stabilisatoren für organisches Material.

**8.** Verwendung von cyclischen $\beta$-Ketocarbonsäure-polyalkylpiperidinylestern I gemäß den Ansprüchen 1

bis 4 als Lichtschutzmittel und Stabilisatoren für Kunststoffe und Lacke.

**9.** Verfahren zum Stabilisieren von organischem Material gegen die Einwirkung von Licht, Sauerstoff und Wärme, dadurch gekennzeichnet, daß man hierzu cyclische $\beta$-Ketocarbonsäure-polyalkylpiperidinylester I gemäß den Ansprüchen 1 bis 4 verwendet.

**10.** Verfahren zum Stabilisieren von Kunststoffen und Lacken gegen die Einwirkung von Licht, Sauerstoff und Wärme, dadurch gekennzeichnet, daß man hierzu cyclische $\beta$-Ketocarbonsäure-polyalkylpiperidinylester I gemäß den Ansprüchen 1 bis 4 verwendet.

Europäisches Patentamt

# EUROPÄISCHER RECHERCHENBERICHT

Nummer der Anmeldung

EP 92 10 3954

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | DE-A-2 656 769 (CIBA-GEIGY) 14. Juli 1977<br>* Seite 46; Beispiel 5 * | 1,3,4 | C07D211/46<br>C08K5/00<br>C08L23/00 |
| Y | * gesamtes Dokument * | 2,5-10 | |
| Y | DE-A-2 849 444 (HOECHST AG) 29. Mai 1980<br>* gesamtes Dokument * | 1-10 | |
| Y | DE-A-2 755 340 (CIBA-GEIGY AG) 29. Juni 1978<br>* gesamtes Dokument * | 1-10 | |
| A | DE-A-2 727 385 (CIBA-GEIGY AG) 29. Dezember 1977<br>* gesamtes Dokument * | 1-10 | |

RECHERCHIERTE SACHGEBIETE (Int. Cl.5 )

C07D
C08K
C08L

Der vorliegende Recherchenbericht wurde für alle Patentansprüche erstellt

| Recherchenort | Abschlußdatum der Recherche | Prüfer |
|---|---|---|
| MUENCHEN | 25 MAI 1992 | HERZ C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)